# DEMANDE DE BREVET EUROPEEN

(11) **EP 0 882 981 A1**
(43) Date de publication de la demande: **09.12.1998**
(21) Numéro de dépôt: 98401346.6
(22) Date de dépôt: 04.06.1998
(51) Int. Cl.: G01N 27/327, G01N 33/543

(54) **Microsystème d'analyse de liquides à cuvette intégrée**

(30) Priorité: 06.06.1997 FR 9707047
(71) Demandeur: COMMISSARIAT A L'ENERGIE ATOMIQUE, 75015 Paris (FR)
(72) Inventeur: Caillat, Patrice, 38130 Echirolles (FR); Ponthenier, Gérard, 38800 Champagnier (FR)
(74) Mandataire: Signore, Robert

(57) **Abrégé**

Microsystème d'analyse à cuvette intégrée comprenant :
- un support de puce (250) présentant au moins une cuvette (252),
- au moins une puce (210) ménagée dans chaque cuvette, la puce comportant des électrodes d'analyse (212) et des électrodes d'adressage (214), et
- des moyens de connexion (258, 260, 262) pour relier les électrodes d'adressage à au moins un appareil électrique extérieur au microsystème.

Conformément à l'invention, les moyens de connexion comportent des bornes de connexion (258) disposées sur le support, à l'extérieur de la cuvette (252), et des liaisons (260, 262) électriques reliant les bornes de connexion (258) aux électrodes d'adressage.

Application à l'analyse de produits chimiques et biologiques.

## Description

### Domaine technique

La présente invention concerne un microsystème d'analyse de liquides, à cuvette intégrée.

L'invention s'inscrit dans le cadre du développement de systèmes d'analyse comprenant une puce électronique avec un circuit intégré spécifique ayant subi une finition particulière qui lui permet d'être utilisé dans un milieu liquide afin d'en déterminer des caractéristiques physiques, chimiques ou biologiques.

A titre d'exemple, un système d'analyse à puce électronique peut être utilisé comme mesureur de glucose, comme dispositif d'analyse sanguine mais aussi comme capteur biologique.

Les capteurs biologiques à circuit électronique sont employés, par exemple, pour la reconnaissance anticorps-antigène ou comme sonde ADN-ADN.

### Etat de la technique antérieure

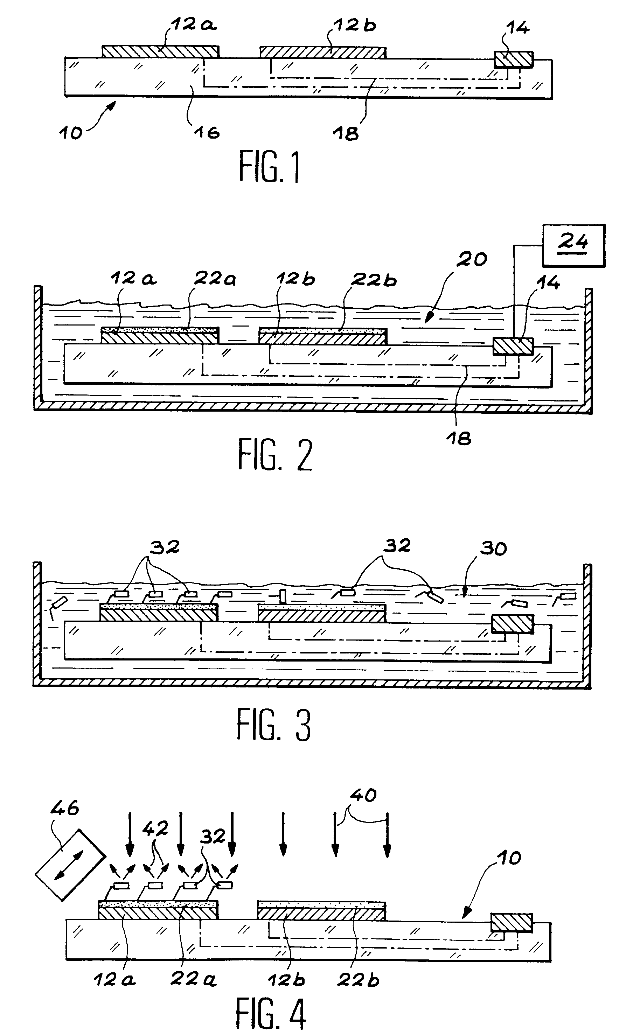

La figure 1 annexée montre en coupe et de façon schématique une puce 10 de capteur biologique tel qu'utilisé pour une reconnaissance anticorps-antigène ou comme sonde ADN-ADN d'un patient.

La puce 10 comporte deux électrodes dites d'analyse 12a et 12b et des électrodes d'adressage 14 dont une seule est visible. Il convient de préciser que les puces pour capteur biologique comportent généralement un grand nombre d'électrodes d'analyse. Ces électrodes peuvent être adressées électriquement par des électrodes d'adressage correspondantes. Toutefois, lorsque le nombre d'électrodes d'analyse est très important, la puce peut comporter un système d'adressage multiplexé permettant d'adresser l'ensemble des électrodes d'analyse à partir d'un nombre réduit d'électrodes d'adressage.

On entend par adressage la mise en liaison électrique d'une ou plusieurs électrodes d'analyse avec une ou plusieurs électrodes dites d'adressage ménagées généralement à la périphérie de la puce. Les électrodes d'adressage permettent d'appliquer ou de mesurer une tension sur les électrodes d'analyse, au moyen d'un appareil extérieur approprié.

Pour des raisons de simplification, seules deux électrodes d'analyse sont représentées sur la puce 10 de la figure 1.

Les électrodes d'analyse 12a et 12b sont réalisées en un métal tel que, par exemple, l'or, ou le platine. Elles sont mutuellement isolées les unes des autres sur une plaque de substrat 16. Des liaisons électriques entre les électrodes d'analyse 12a, 12b et les électrodes d'adressage sont ménagées dans le substrat 16 et sont très schématiquement indiquées avec la référence 18.

Une puce, telle que représentée à la figure 1, doit être configurée pour une utilisation particulière et les électrodes d'analyse sont à cet effet rendues fonctionnelles en les garnissant de molécules-sondes ou en recouvrant leur surface par un dépôt de réactif approprié.

Le dépôt de produits réactifs ou le greffage de molécules-sondes sur les électrodes est réalisé, en général, par électrodéposition.

Les réactifs ou molécules-sondes déposés sur les électrodes permettent un appariement sélectif avec des molécules spécifiques d'une substance à analyser. Ces molécules sont désignées par "molécules-cibles" dans la suite du texte.

La figure 2 montre la puce 10 plongée dans un bain d'électrolyte 20. On entend par bain d'électrolyte un bain approprié pour déposer, par électrochimie, un réactif sur les électrodes, ou un bain dans lequel sont diluées des molécules-sondes devant être fixées par électrodéposition sur les électrodes.

L'application sélective d'une tension de polarisation entre des électrodes d'analyse sélectionnées et une électrode de référence permet d'y fixer le produit réactif ou les molécules-sondes. La tension est appliquée aux électrodes au moyen d'un générateur extérieur 24 connecté aux électrodes d'adressage 14.

Les molécules-sondes sont fixées sur les électrodes d'analyse, par exemple, au moyen de polymères conducteurs de type polypyrrol ou polyaniline qui sont des porteurs de molécules-sondes.

La puce peut subir plusieurs étapes de dépôt électrochimique en étant trempée dans différents bains. Ainsi, différentes électrodes de la puce peuvent être recouvertes de différents réactifs, ou molécules-sondes, sensibles à différents composés des substances à analyser.

Sur la figure 2, on considère que les deux électrodes 12a et 12b sont respectivement (et successivement) recouvertes de réactifs, ou de molécules-sondes, différents 22a, 22b.

A l'issue du garnissage des électrodes avec les réactifs, de molécules-sondes, la puce est prête pour être utilisée afin d'analyser une substance désignée par analyte dans la suite de la description.

Comme le montre la figure 3, la puce 10 est déconnectée du générateur et est plongée dans un bain 30 contenant l'analyte.

Ce bain contient, par exemple, des molécules-cibles 32 qui s'apparient ou réagissent avec le réactif ou les molécules-sondes 22a préalablement déposés sur la première électrode 12a. Pour des raisons de clarté, les molécules-cibles 32 sont représentées schématiquement et de façon grossie.

Les molécules-cibles 32 n'interagissent cependant pas avec la deuxième électrode 12b dont le revêtement réactif, ou les molécules-sondes, ne sont pas compatibles.

Après avoir été extraite du bain d'analyte 30, la puce est analysée afin de déterminer les électrodes pour lesquelles une réaction ou un appariement ont eu lieu.

Dans l'exemple décrit à la figure 4, on analyse la puce 10 par une méthode de détection par fluorescence. Une telle méthode est particulièrement adaptée lorsque les molécules-cibles 32 sont marquées par un produit de marquage fluorescent dit fluophore.

Toutefois, d'autres méthodes d'analyse telles que des méthodes de mesure électrique par impédancemétrie, de mesure par microbalance, des mesures optiques par changement d'indice de réfraction, et des méthodes d'analyse par marquage radioactif sont également envisageables.

Comme le montre la figure 4, l'ensemble de la puce 10 est soumise à un rayonnement lumineux 40 d'une première longueur d'onde et provenant d'une source lumineuse non représentée.

Les molécules-cibles marquées absorbent le rayonnement lumineux 40 et émettent un rayonnement lumineux 42 avec une deuxième longueur d'onde caractéristique, différente de la première longueur d'onde.

Un système de détection 46, sensible à la deuxième longueur d'onde permet de détecter la lumière réémise depuis les électrodes portant les molécules-cibles marquées par le produit fluorescent. Il est ainsi possible, en connaissant la nature des réactifs ou des molécules-sondes préalablement déposés sur chaque électrode de déterminer les composants de l'analyte qui s'y sont fixés.

Une difficulté rencontrée lors de la mise en oeuvre de la méthode d'analyse illustrée aux figures 1 à 4 est liée à la grande quantité de liquide requise pour former le bain d'électrolyte (figure 2), ainsi qu'à la grande quantité d'analyte nécessaire pour y plonger la puce 10 (figure 3).

Une autre difficulté est la mise en place et le retrait des tranches ou des puces dans un porte-échantillon. Leur connexion, constitue une opération délicate et fastidieuse qui doit être faite au moins deux fois : pour le garnissage des électrodes et pour l'analyse. De plus, l'utilisateur des puces ne dispose pas nécessairement de l'équipement du porte-échantillon et ne peut pas envisager une reconfiguration individuelle des puces par un dépôt complémentaire d'autres réactifs que ceux qui y sont déposés à l'origine.

Se posent également des problèmes d'altération du connecteur qui se trouve en contact avec la tranche et par conséquent avec le liquide contenu dans le porte-échantillon.

Le porte-échantillon pourrait éventuellement être adapté pour contenir une unique puce et le liquide à analyser. Cependant, l'utilisateur constate que la mise en place et le retrait de puces du porte-échantillon est peu compatible avec des analyses effectuées sur des grandes séries de puces.

Enfin, lorsque les électrodes d'adressage sont reliées à un appareil de mesure pour effectuer une analyse qualitative des composés ayant réagi avec les électrodes de la puce, il apparaît que le liquide à analyser vient mouiller les électrodes d'adressage et/ou le connecteur et risque de fausser les mesures.

### Exposé de l'invention

L'invention a pour but de proposer un microsystème d'analyse permettant d'éviter les difficultés énumérées ci-dessus.

Un but est en particulier de proposer un microsystème à faible coût, d'une utilisation aisée et nécessitant des quantités faibles d'électrolyte ou d'analyte pour effectuer les analyses décrites précédemment.

Un but est aussi de proposer un système facilement reconfigurable par l'utilisateur et se présentant sous une forme facilitant les opérations d'analyse.

Un but est enfin de proposer un système d'analyse fiable dans lequel le liquide à analyser ne risque pas de venir en contact avec des connecteurs reliés à des appareils extérieurs.

Pour atteindre ces buts, l'invention a plus précisément pour objet un microsystème d'analyse à cuvette intégrée comprenant :
- un support de puce présentant au moins une cuvette,
- au moins une puce ménagée dans la cuvette du support, la puce comportant des électrodes d'analyse et des électrodes d'adressage, et
- des moyens de connexion pour relier les électrodes d'adressage à au moins un appareil électrique extérieur au microsystème.

Conformément à l'invention, les moyens de connexion comportent des bornes de connexion disposées sur le support, à l'extérieur de la cuvette, et des liaisons électriques reliant les bornes de connexion aux électrodes d'adressage.

Grâce à l'invention, et notamment au fait que les bornes de connexion sont situées sur le support et non sur la puce elle-même, l'ensemble formé par la puce et le support est très ergonomique et permet une connexion aisée vers des appareils extérieurs. En particulier, une telle conception permet à un utilisateur de "reconfigurer" une puce en effectuant de nouveaux dépôts de réactifs sur les puces, sans que pour cela un équipement particulier soit nécessaire et sans utiliser des bains d'électrolyte nécessitant de grandes quantités de liquide. De plus, l'électrolyte ou l'analyte contenu dans la cuvette ne risque par de venir en contact avec les bornes de connexion ou les connecteurs des appareils extérieurs qui y sont reliés.

Les appareils électriques extérieurs peuvent être soit des générateurs de tension pour appliquer des potentiels électriques prédéterminés aux électrodes de la puce, soit des appareils de mesure électriques permettant de mesurer des grandeurs électriques, telles qu'une conductivité, entre des électrodes de la puce.

Selon une conception particulière du microsystème, la puce peut comporter un substrat présentant les électrodes d'analyse et d'adressage, le substrat étant fixé à demeure dans la cuvette du support de puce.

Une telle conception est particulièrement avantageuse car elle permet une fabrication séparée en grande série des puces et du support.

En particulier, un grand nombre de puces peut être réalisé collectivement sur une même tranche de semi-conducteur qui est finalement découpée. Les puces sont alors individuellement intégrées à un support pour constituer un microsystème d'analyse conforme à l'invention.

Selon une autre conception, la puce peut être directement réalisée dans le support de puce. Ainsi, le support de puce constitue également un substrat de support pour les électrodes d'analyse et d'adressage.

Selon d'autres aspects de l'invention, le microsystème peut comporter des moyens de chauffage de la puce intégrés sous la puce, soit directement dans le substrat de la puce, soit dans le support de puce.

Ces moyens de chauffage, associés éventuellement à un thermocouple permettent de contrôler et piloter précisément des températures de dépôt de réactifs ou des températures d'analyse sur les électrodes.

Selon un autre aspect avantageux, la taille et le volume de la cuvette peuvent être ajustés en fonction de la puce qui y est logée et des applications envisagées. Ainsi, une juste quantité d'électrolyte ou d'analyte est contenue dans la cuvette.

Conformément à une réalisation particulière du support de puce, celui-ci peut comporter un premier substrat formant un fond de la cuvette et un deuxième substrat disposé sur le premier substrat et formant des parois latérales de la cuvette

Selon une variante, la cuvette peut également être gravée directement dans un substrat massif.

D'autres caractéristiques et avantages de l'invention ressortiront mieux de la description qui va suivre, en référence aux figures des dessins annexés. Cette description est donnée à titre purement illustratif et non limitatif.

### Brève description des figures

- La figure 1, déjà décrite, est une coupe schématique d'une puce de capteur biologique.
- La figure 2, déjà décrite, est une coupe schématique de la puce de la figure 1 plongée dans un bain d'électrolyte.
- La figure 3, déjà décrite, est une coupe schématique de la puce de la figure 1, plongée dans un bain d'analyte.
- La figure 4, déjà décrite, est une coupe schématique de la puce de la figure 1, soumise à une analyse par fluorescence.

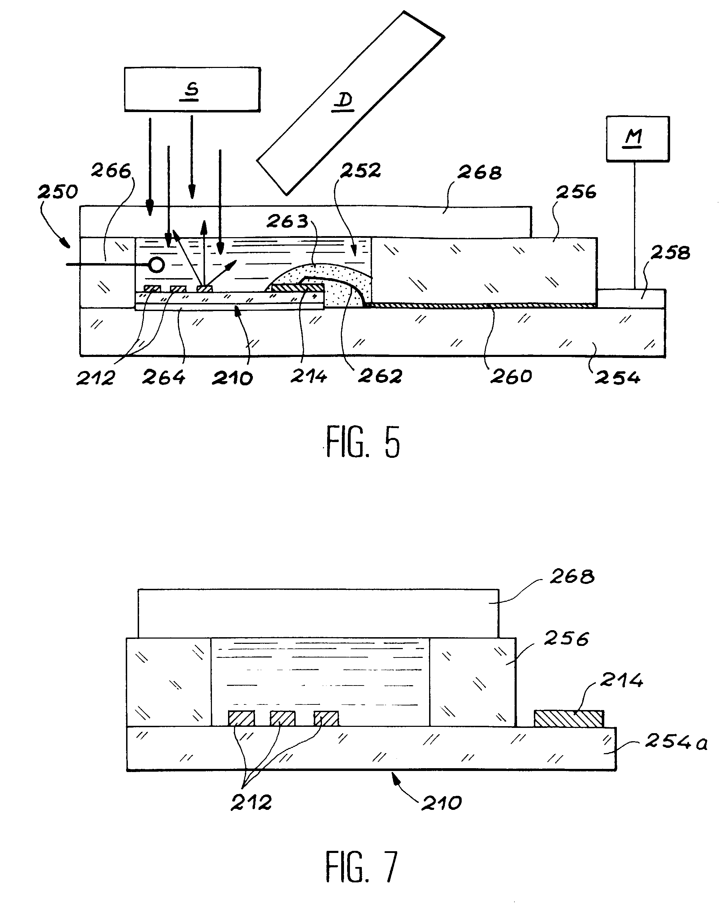

- La figure 5 est une coupe schématique d'un microsystème d'analyse de liquides conforme à l'invention.

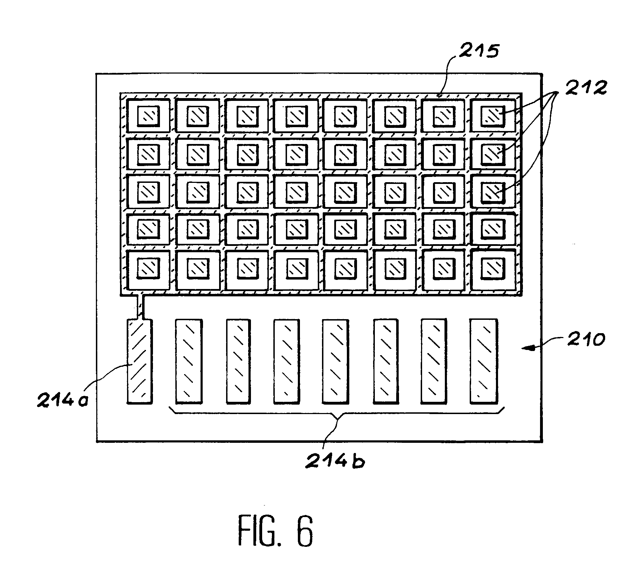

- La figure 6 est une vue de dessus à plus grande échelle d'une puce pouvant être intégrée dans le microsystème d'analyse de la figue 5.
- La figure 7 est une coupe schématique d'un microsystème d'analyse de liquides, conforme à l'invention, constituant une variante du microsystème de la figure 5.

### Description détaillée de modes de mise en oeuvre de l'invention

Un microsystème d'analyse conforme à l'invention peut comporter plusieurs cuvettes contenant des puces électroniques. Toutefois, selon une réalisation préférée, chaque microsystème ne présente qu'une seule cuvette avec une seule puce.

Ainsi, le microsystème d'analyse représenté à la figure 5 comprend un support de puce 250 présentant une unique cuvette 252. La cuvette 252 contient une puce électronique 210, telle que, par exemple, une puce de capteur biologique ou chimique.

Le support de puce 250 comprend pour l'essentiel un premier substrat 254 formant un fond de la cuvette 252 et un deuxième substrat 256 rapporté (par exemple par collage) sur le premier substrat et définissant la ou les parois latérales de la cuvette. Les premier et deuxième substrats sont de préférence des substrats isolants électriques en un matériau tel qu'un matériau composite fibres de verre/résine époxy.

Le premier substrat 254 comporte en une extrémité située en dehors de la cuvette un ensemble de bornes de connexion 258. Les bornes 258, séparées de la cuvette par le deuxième substrat, sont reliées électriquement à des électrodes d'adressage 214 de la puce 210 ménagée dans la cuvette 252. La liaison électrique entre les bornes et les électrodes d'adressage est réalisée, par exemple, par des pistes conductrices 260, passant entre les premier et deuxième substrats, et par des fils de connexion 262 reliant les pistes conductrices 260 aux électrodes d'adressage 214 correspondantes.

Pour protéger et isoler les électrodes d'adressage 214 et les fils de connexion 262 des liquides versés dans la cuvette 252, ceux-ci sont enrobés, par exemple, d'une couche isolante 263 de matériau polymère.

La référence 264 indique une résistance chauffante disposées sous la puce 214. Cette résistance, qui peut être alimentée électriquement depuis l'extérieur du support, permet de piloter la température de la puce 210 et des électrodes d'analyse 212 de la puce.

Le contrôle de la température peut être effectué par un thermocouple 266 s'étendant dans la cuvette pour mesurer la température d'un liquide qu'elle comprend.

La cuvette 252 du support peut être emplie d'un électrolyte ou d'une solution contenant des molécules-sondes afin d'en garnir les électrodes d'analyse 212 de la puce de la façon décrite précédemment.

Lorsque cette opération est achevée, l'électrolyte peut être remplacé par l'analyte.

Avantageusement, un couvercle 268 prenant appui sur le deuxième substrat 256 peut être prévu pour fermer hermétiquement la cuvette 252 et y conserver l'analyte pendant son analyse. Cette caractéristique facilite la manipulation du microsystème.

Lorsque la méthode d'analyse est une méthode par mesure de fluorescence, telle que décrite précédemment, le couvercle 268 peut avantageusement être constitué par une lame de verre qui laisse passer un rayonnement incident provenant d'une source lumineuse S et qui laisse passer le rayonnement de fluorescence vers un détecteur D.

L'analyse des propriétés ou de la composition de l'analyte peut également se faire par des mesures électriques de conductibilité en reliant un appareil de mesure approprié M aux bornes de connexion 258.

La figure 6 montre un exemple de puce 210 pouvant être utilisée dans le microsystème de l'invention.

La puce 210 comporte, sur un substrat, une pluralité d'électrodes d'analyse 212, réparties en lignes et en colonnes, et éventuellement une contre-électrode 215 sous la forme d'une grille entourant les électrodes d'analyse. Les électrodes d'analyse 212 et la contre-électrode sont en un matériau tel que l'or ou le platine par exemple.

La puce comporte également une pluralité d'électrodes d'adressage dont une première électrode d'adressage 214a est reliée directement à la contre électrode 215. Les autres électrodes d'adressage 214b sont connectées aux électrodes d'analyse 212, par exemple quand le nombre d'électrodes est important, par l'intermédiaire d'un multiplexeur-démultiplexeur intégré dans la puce et non représenté sur la figure.

Pour garnir une électrode d'analyse d'un réactif par électrodéposition, une tension est appliquée entre cette électrode et la contre-électrode par un adressage approprié des électrodes d'adressage.

De la même façon, lors d'une étape d'analyse, un courant de mesure peut sélectivement être mesuré entre une électrode d'analyse donnée et la contre-électrode.

La puce de la figure 6 peut être fixée à demeure dans le support de puce 250 décrit précédemment (figure 5), par exemple, en collant le substrat de la puce 210 dans le fond de la cuvette, par une face ne comportant pas les électrodes.

La figure 7 montre une autre réalisation du microsystème de l'invention, constituant une variante de la réalisation décrite ci-dessus.

Selon cette variante, la puce 210 comporte un substrat 254a qui n'est pas collé dans le fond de la cuvette, mais qui constitue directement le premier substrat, et qui forme donc le fond de la cuvette.

Ainsi, le deuxième substrat 256, qui définit les parois latérales de la cuvette et qui est rapporté sur le substrat 254a, est en fait rapporté directement sur la puce 210.

Par analogie avec la figure 5, les références 212, 214 et 268 désignent respectivement les électrodes d'analyse, les électrodes d'adressage et le couvercle de la cuvette.

## Revendications

1. Microsystème d'analyse à cuvette intégrée, caractérisé en ce qu'il comprend :
- un support de puce (250) présentant au moins une cuvette (252),
- au moins une puce (210) ménagée dans la cuvette du support, la puce comportant des électrodes d'analyse (212) et des électrodes d'adressage (214), et
- des moyens de connexion (258, 260, 262) pour relier les électrodes d'adressage à au moins un appareil électrique extérieur au microsystème,
et dans lequel les moyens de connexion comportent des bornes de connexion (258) disposées sur le support, à l'extérieur de la cuvette (252), et des liaisons (260, 262) électriques reliant les bornes de connexion (258) aux électrodes d'adressage.

2. Microsystème selon la revendication 1, dans lequel la puce comporte un substrat (210) présentant les électrodes (212, 214) d'analyse et d'adressage, le substrat étant fixé à demeure dans la cuvette du support de puce.

3. Microsystème selon la revendication 1, dans lequel la puce est réalisée directement sur le support de puce.

4. Microsystème selon la revendication 1, comportant des moyens (264) de chauffage de la puce.

5. Microsystème selon la revendication 4, dans lequel les moyens de chauffage comportent une résistance électrique intégrée dans la puce.

6. Microsystème selon la revendication 4, dans lequel les moyens de chauffage comportent une résistance électrique (264) ménagée dans le support de puce, sous la puce.

7. Microsystème selon la revendication 1, dans lequel le support de puce comporte un thermocouple (266).

8. Microsystème selon la revendication 1, présentant dans la cuvette un enrobage (263) d'isolation des électrodes d'adressage (214) et des liaisons électriques (262).

9. Microsystème selon la revendication 1 dans lequel la cuvette présente un volume déterminé en fonction d'une quantité de liquide de traitement requise par la puce.

10. Microsystème selon la revendication 1, dans lequel le support de puce comporte un premier substrat (254) formant un fond de la cuvette et un deuxième substrat (256) disposé sur le premier substrat et formant des parois latérales de la cuvette.

11. Microsystème selon la revendication 10 comprenant une lame de verre (268) disposée sur le second substrat (256) et recouvrant la cuvette.

12. Microsystème selon la revendication 10, dans lequel la puce (210) comporte un substrat (254a), ledit substrat constituant le premier substrat formant le fond de la cuvette.

13. Microsystème selon la revendication 1, comprenant un substrat massif et dans lequel la cuvette est gravée dans le substrat massif.
